# EUROPÄISCHE PATENTANMELDUNG

(11) **EP 2 157 569 A2**
(43) Veröffentlichungstag der Anmeldung: **24.02.2010**
(21) Anmeldenummer: 09167534.8
(22) Anmeldetag: 10.08.2009
(51) Int. Cl.: G10K 11/26

(54) **Verfahren und Stoßwellenkopf zum Erzeugen von fokussierten Ultraschall-Stoßwellen**

(30) Priorität: 18.08.2008 DE 102008038214
(71) Anmelder: Siemens Aktiengesellschaft, 80333 München (DE)
(72) Erfinder: Fehre, Jens, 91353 Hausen (DE); Nanke, Ralf, 91077 Neunkirchen am Brand (DE); Rohwedder, Arnim, 90766 Fürth (DE)

(57) **Zusammenfassung**

Die Erfindung bezieht sich auf ein Verfahren zum Erzeugen von Ultraschall-Stoßwellen (2a-d) sowie einem nach diesem Verfahrenen betriebenen Stoßwellenkopf (30). Mit einer in dem Stoßwellenkopf (30) angeordneten Stoßwellenquelle (32) werden zeitlich nacheinander eine Mehrzahl von jeweils in einem Fokus (Fa-d) fokussierten Ultraschall-Stoßwellen (2a-d) erzeugt, die sich entlang unterschiedlicher Schallachsen (za-d) ausbreiten und eine den jeweiligen Fokus (Fa-d) umgebende Nutzzone (8a-d) mit einer Fokalbreite (B) aufweisen, wobei die Foki (Fa-d) voneinander beabstandet in vorgegebenen Positionen derart angeordnet sind, dass sich die Fokusvolumina (8a-d) der Ultraschall-Stoßwellen (2a-d) im Bereich der Nutzzonen (8a-d) derart überlagern, dass sich ein zusammenhängender Nutzbereich (20) ergibt, dessen effektive Fokalbreite (ΣB) größer ist als die Fokalbreite (B) der Nutzzonen (8a-d) der einzelnen Ultraschall-Stoßwellen (2a-d).

## Beschreibung

Die Erfindung bezieht sich auf ein Verfahren zum Erzeugen von fokussierten Ultraschall-Stoßwellen für die extrakorporale Stoßwellenlithotripsie. Außerdem bezieht sich die Erfindung auf einen nach diesem Verfahren betriebenen Stoßwellenkopf.

Die extrakorporale Stoßwellenlithotripsie nutzt die therapeutische Wirkung von fokussierten Ultraschall-Stoßwellen, um im Körper eines Lebewesens befindliche Konkremente, beispielsweise in der Niere befindliche Harnsteine zu zertrümmern. Durch die Fokussierung der Ultraschall-Stoßwelle auf ein Zielgebiet, in dem sich das Konkrement befindet, wird außerdem eine Schädigung des das Konkrement umgebenden Gewebes, in der Regel die Bildung von Hämatomen, minimiert.

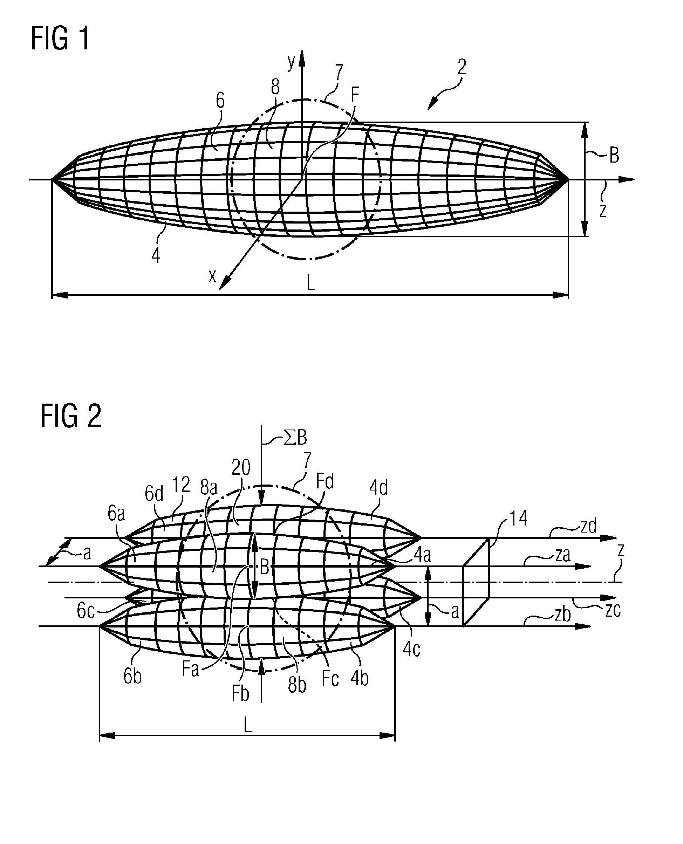

In Fig. 1 ist das typische Schallfeld einer in der Stoßwellenlithotripsie üblicherweise verwendeten fokussierten Ultraschall-Stoßwelle 2 im Bereich des Fokus F dargestellt. Eingezeichnet ist eine Einhüllende 4, die durch die Punkte gebildet ist, an denen die Druckamplitude der Ultraschall-Stoßwelle 2 gegenüber der Druckamplitude im Fokus F um 6 dB abgesunken ist. Als Fokus F der Ultraschall-Stoßwelle 2 wird der Punkt bezeichnet, an dem der maximale positive Schalldruck im Schallfeld auftritt. Die Länge dieser Einhüllenden 4 in Richtung der Bündel- oder Schallachse z wird als Fokalausdehnung L bezeichnet. Als Fokalbreite B wird die größte Breite der Einhüllenden um den Fokus F in der zur oder Schallachse z senkrechten x,y-Ebene bezeichnet. Im innerhalb der Einhüllenden 4 befindlichen Volumen - das sogenannte Fokusvolumen 6 - tritt eine maximale Wirkung der Ultraschall-Stoßwelle 2 auf.

Der Figur ist nun zu entnehmen, dass das Fokusvolumen 6 einer fokussierten Ultraschall-Stoßwelle 2 etwa die Form einer in Richtung der Schallachse z sich erstreckenden Zigarre hat. In die Figur 1 ist außerdem beispielhaft in Gestalt einer Kugel ein Therapiegebiet 7 eingezeichnet, das ein zu zertrümmerndes Konkrement umschließt. Das innerhalb des Therapiegebietes 7 befindliche Fokusvolumen 6 ist die therapeutisch genutzte Zone der Ultraschall-Stoßwelle 2 und wird im folgenden als Nutzzone 8 bezeichnet. Diese Nutzzone 8 ist ein den Fokus F umgebendes Gebiet, dessen Ausdehnung in Richtung der Schallachse z annähernd der Fokalbreite B entspricht. Die Fokalausdehnung L in Richtung der Schallachse z ist um den Faktor 6 bis 10 größer ist als die Fokalbreite B (größte Lateralausdehnung der Nutzzone 8). Ursache hierfür ist die Wellennatur der Ultraschall-Stoßwelle 2.

Im Hinblick auf die kugelförmige Gestalt des Therapiegebietes 7 wäre dementsprechend zum Zertrümmern des Konkrements eine Ultraschall-Stoßwelle ideal, deren Fokusvolumen 4 ebenfalls eine kugelförmige Gestalt hat. Eine fokussierte Ultraschall-Stoßwelle 2 mit insgesamt kugelförmigem Fokusvolumen kann jedoch nur von einem Stoßwellengenerator mit kugelförmiger Apertur erzeugt werden. Dieser müsste dann das Therapiegebiet und zwangsläufig auch den Patienten umgeben. Abgesehen vom apparativen Aufwand und der Befindlichkeit des Patienten steht dem auch die akustische Inhomogenität des menschlichen Körpers entgegen. Träfen Segmente der fokussierten Ultraschall-Stoßwelle auf Knochen oder luftgefüllte Gebiete (Lunge) würden diese reflektiert und absorbiert und würden dann bei der Bildung eines kugelförmigen Fokusvolumens fehlen. Mit anderen Worten: Auch in diesem Fall wäre die effektive Apertur nicht mehr kugelförmig.

Da sich unterschiedliche Schallwege durch menschliches Gewebe durch entsprechend unterschiedliche Absorption ähnlich auswirken, stehen für die Stoßwellenlithotripsie in der Praxis nur relativ kleine Schallfenster zur Verfügung. Aus diesem Grund haben sich nur, im folgenden als Stoßwellenkopf bezeichnete Ultraschall-Stoßwellenquellen mit sehr begrenzten Aperturen durchsetzen können, so dass die in der Praxis verwendeten Fokusvolumina die vorstehend erläuterte Gestalt haben. Die Fokalbreite B der Ultraschall-Stoßwelle 2 ist dabei so bemessen, dass sie annähernd dem typischen Durchmesser der in der Stoßwellenlithotripsie therapeutisch behandelbaren Konkremente - etwa 10mm - entspricht. Dies führt dazu, dass sich in Richtung der Bündelachse z bis zu 5cm vor und hinter dem Fokus F ein Gebiet mit einer hohen Druckamplitude befindet, so dass in der Praxis eine Schädigung des vor oder hinter dem Konkrement liegenden Gewebes möglich ist.

Der Erfindung liegt daher die Aufgabe zugrunde, ein insbesondere für die Stoßwellen-Lithotripsie geeignetes Verfahren zum Erzeugen fokussierter Ultraschall-Stoßwellen anzugeben, mit dem das Risiko einer Beschädigung des ein Konkrement umgebenden Gewebes gegenüber bekannten Verfahren verringert werden kann. Außerdem liegt der Erfindung die Aufgabe zugrunde, einen zum Durchführen diesen Verfahrens geeigneten Stoßwellenkopf anzugeben.

Hinsichtlich des Verfahrens wird die genannte Aufgabe gelöst mit einem Verfahren mit den Merkmalen des Patentanspruches 1. Bei diesem Verfahren werden mit einer in einem Stoßwellenkopf angeordneten Stoßwellenquelle zeitlich nacheinander mehrere fokussierte Ultraschall-Stoßwellen erzeugt, die sich entlang unterschiedlicher Schallachsen ausbreiten und eine den jeweiligen Fokus umgebende Nutzzone mit einer Fokalbreite aufweisen, wobei die Foki voneinander beabstandet in vorgegebenen Positionen relativ zueinander derart angeordnet sind, dass sich die Fokusvolumina der Ultraschall-Stoßwellen im Bereich der Nutzzonen derart überlagern, dass sich ein zusammenhängender Nutzbereich ergibt, dessen Fokalbreite größer ist als die Fokalbreite der einzelnen Ultraschall-Stoßwellen.

Mit anderen Worten: Die Nutzzonen der einzelnen Ultraschall-Stoßwellen überlagern sich nicht vollständig sondern jeweils nur in einem Randgebiet, so dass ein effektiver zusammenhängender, d. h. lückenloser Nutzbereich entsteht, in dem die Schalldruckamplitude größer ist als an der das Fokusvolumen begrenzenden Einhüllenden, und dessen Volumen größer ist als das Volumen der Nutzzone einer einzelnen Ultraschall-Stoßwelle. Da sich die Foki in vorgegebenen Positionen relativ zueinander befinden, erfolgt eine gezielte Überlagerung der einzelne Fokusvolumina und damit reproduzierbare Form des effektiven Fokusvolumens.

Durch eine solche Überlagerung kann mit zeitlich aufeinanderfolgenden Ultraschall-Stoßwellen ein relativ großer effektiver zentraler Nutzbereich auch dann erzeugt werden, wenn die einzelnen Fokusvolumina sowohl in Richtung der Schallachse als auch quer dazu nur eine kleine Ausdehnung, d. h. eine kleine Fokalausdehnung und eine dementsprechend kleine Fokalbreite haben.

Die Erfindung beruht dabei auf der Überlegung, dass die Fokalbreite und die Fokalausdehnung durch Erhöhung der Schwerpunktsfrequenz der Ultraschall-Stoßwelle nahezu indirekt proportional zu dieser verringert werden kann. So bewirkt eine Verdopplung der Schwerpunktsfrequenz annähernd eine Halbierung von Fokalausdehnung und Fokalbreite. Bei gleichbleibendem Spitzenschalldruck im Fokus ist es durch Anordnung einer Mehrzahl kleinerer Fokusvolumina und deren Überlagerung in der Umgebung der Foki möglich, ein Fokusvolumen mit verkürzter Fokalausdehnung zu erzeugen, dessen Fokalbreite der Fokalbreite einer mit der halben Schwerpunktsfrequenz erzeugten Ultraschall-Stoßwelle entspricht. Mit anderen Worten: Das Therapiegebiet, d. h. das Gebiet, in dem die Ultraschall-Stoßwelle ihre therapeutische Wirkung entfalten soll, wird nacheinander mit Ultraschall-Stoßwellen mit kleiner Fokalbreite gescannt, um auf diese Weise eine Vergrößerung der Fokalbreite bei unveränderter Tiefenausdehnung zu erzielen.

Ein Nutzbereich mit in Umfangsrichtung um eine mittlere Schallachse möglichst konstanter Fokalbreite wird erzielt, wenn die Schallachsen symmetrisch um eine die mittlere Schallachse definierende Systemachse des Stoßwellenkopfes angeordnet sind.

Die Foki der Ultraschall-Stoßwellen befinden sich zumindest annähernd in einer zur Systemachse senkrechten Ebene, wobei die Foki vorzugsweise auf einer Kreisbahn um die Systemachse angeordnet sind. Auf diese Weise ist bei minimaler Fokalausdehnung eine große effektive Fokalbreite quer zur mittleren Schallachse erreichbar.

Wenn die Schallachsen zueinander parallel sind, ist es möglich, die Fokusvolumina von entlang benachbarter Schallachsen sich ausbreitender Ultraschall-Stoßwellen derart einzustellen, dass sich diese ausschließlich in den Nutzzonen überlappen. Auf diese Weise ist eine Schädigung des Gewebes im Bereichen außerhalb der Nutzzonen zusätzlich verringert. Alternativ hierzu können die Schallachsen auch zueinander geneigt sein.

Zum Erzeugen der sich entlang unterschiedlicher Schallachsen ausbreitenden Ultraschall-Stoßwellen wird vorzugsweise die Stoßwellenquelle innerhalb des Stoßwellenkopfes unterschiedlich positioniert Bei dieser Vorgehensweise ist eine Bewegung des Stoßwellenkopfes nicht erforderlich, so dass dieser nicht nach jedem Schuss neu positioniert und blasenfrei am Körper des Patienten angekoppelt werden muss.

Alternativ hierzu können die verschiedenen Schallachsen auch dadurch erzeugt werden, dass einem Stoßwellengenerator die Lage in Richtung der Schallachse der Ultraschall-Stoßwellen beeinflussende Komponente nachgeordnet ist, die zum Erzeugen der entlang verschiedener Schallachsen sich ausbreitenden Ultraschall-Stoßwellen relativ zum ruhenden Stoßwellengenerator unterschiedlich positioniert werden.

Hinsichtlich des Stoßwellenkopfes wird die Aufgabe gemäß der Erfindung gelöst mit einem Stoßwellenkopf mit den Merkmalen des Patentanspruches 10, deren Vorteile ebenso wie die Vorteile der dem Patentanspruch nachgeordneten Unteransprüche sinngemäß den Vorteilen der jeweils zugehörigen Verfahrensansprüche entsprechen.

Zur weiteren Erläuterung der Erfindung wird auf die Ausführungsbeispiele der Zeichnung verwiesen. Es zeigen:
- Fig. 1: die typische Form eines fokussierten Schallfeldes, wie es von einem in der Lithotripsie verwendeten Stoßwellenkopf erzeugt wird,
- Fig. 2: ein Ausführungsbeispiel für die Form eines gemäß der vorliegenden Erfindung erzeugten Schallfeldes,
- Fig. 3 bis 5: jeweils Ausführungsbeispiele eines erfindungsgemäßen Stoßwellenkopfes in einer grob schematisierten Prinzipdarstellung,
- Fig. 6: ein zu Fig. 3 korrespondierendes Ausführungsbeispiel einer Stoßwellenquelle gemäß der Erfindung in einem schematischen Längsschnitt,
- Fig. 7: einen dem Prinzipbild der Fig. 5 entsprechendes Ausführungsbeispiel eines Stoßwellenkopfes ebenfalls in einem Längsschnitt.

Fig. 2 zeigt das effektive Fokusvolumen 12 eines aus vier zeitlich aufeinanderfolgenden Ultraschall-Stoßwellen 2a bis 2d durch Überlagerung zusammengesetzten Schallfeldes. In diesem Beispiel breiten sich die Ultraschall-Stoßwellen 2a bis 2d jeweils parallel zueinander entlang voneinander beabstandeter Schallachsen za bis zd aus, die symmetrisch um eine mittlere Schallachse z verteilt sind. Die Schallachsen za bis zd haben zur jeweils nächstliegenden benachbarten Schallachse den Abstand a, der kleiner oder höchstens gleich der Fokalbreite B der Ultraschall-Stoßwellen 2a bis 2d ist. Die zu den Ultraschall-Stoßwellen 2a bis 2d gehörenden Foki Fa bis Fd bilden die Eckpunkte eines Quadrates 14 und spannen eine senkrecht zur mittleren Schallachse z orientierte Ebene auf. Der Figur ist zu entnehmen, dass sich die Fokusvolumina 6a bis 6d der Ultraschall-Stoßwellen 2a bis 2d im Bereich ihrer Nutzzonen 8a bis 8d überlagern, so dass sich ein zusammenhängender Nutzbereich 20 ergibt, dessen Form und Ausdehnung annähernd der Form und Ausdehnung der in Fig. 1 dargestellten Nutzzone 8 entspricht. Durch eine Erhöhung der Schwerpunktsfrequenz der Ultraschall-Stoßwellen 2a bis 2d bei gleichbleibender Apertur der Stoßwellenquelle ist die effektive Fokalbreite ΣB größer als die Fokalbreite B der Nutzzonen 6a bis 6d der einzelnen Ultraschall-Stoßwellen 2a bis 2d und entspricht annähernd der Fokalbreite B der Nutzzone 8 der Ultraschall-Stoßwelle 2, obwohl die Fokalausdehnung L des aus den Ultraschall-Stoßwellen 2a bis 2d zusammengesetzten Schallfeldes deutlich kleiner als die Fokalausdehnung L der Ultraschall-Stoßwelle 2 (Fig. 1) ist. Dabei wird ausgenutzt, dass das Fokusvolumen 6a bis 6d jeder einzelnen Ultraschall-Stoßwelle 2a bis 2d in den jeweiligen Nutzzonen 8a bis 8d die größte Breite aufweist, und sich die Fokusvolumina 6a bis 6d der einzelnen Ultraschall-Stoßwellen 2a bis 2d ausschließlich in einem innerhalb der Nutzzonen 8a bis 8d liegendem Gebiet überlagern. Mit dem auf diese Weise erzeugten zusammengesetzten Nutzbereich 20 wird somit das in Fig. 1 dargestellte Therapiegebiet 7 effektiv in der annähernd gleichen Weise abgedeckt wie mit der Ultraschall-Stoßwelle 2.

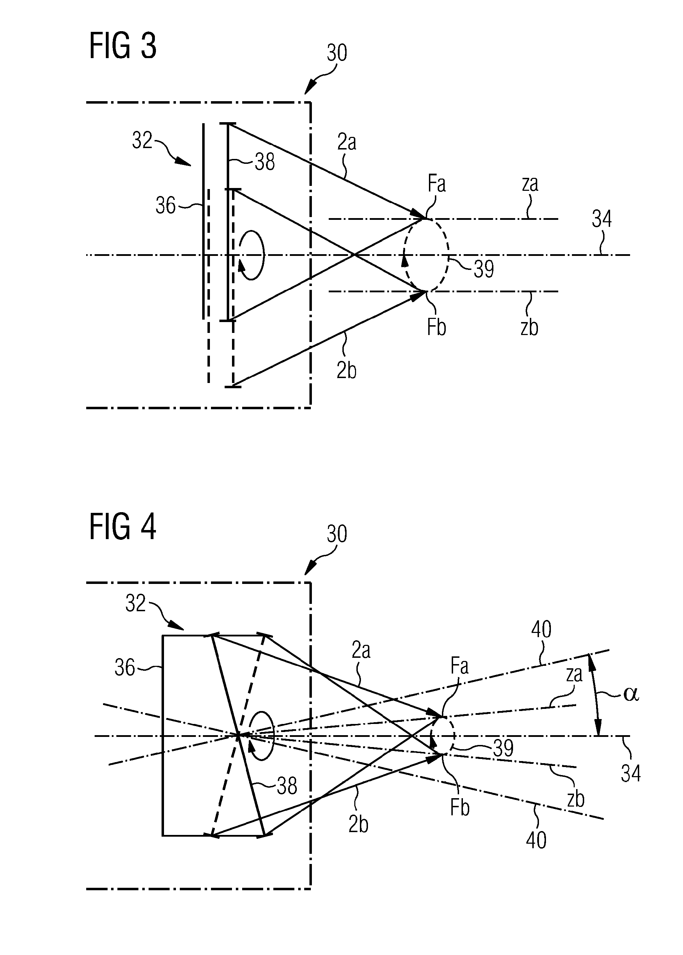

Das in Fig. 2 dargestellte Schallfeld kann gemäß Fig. 3 dadurch erzeugt werden, dass in einem Stoßwellenkopf 30 eine Stoßwellenquelle 32 variabel positionierbar, im Ausführungsbeispiel drehbar um eine Systemachse 34 gelagert ist. Diese Systemachse 34 entspricht dann der mittleren Schallachse z der Fig. 2. Im Beispiel ist eine Stoßwellenquelle 32 veranschaulicht, die aus einem ebenen Stoßwellengenerator 36 aufgebaut ist, der eine ebene Ultraschall-Stoßwelle emittiert, aus der durch eine akustische Linse 38 eine in einem Fokus Fa, Fb fokussierte Ultraschall-Stoßwelle 2a, b erzeugt wird. Akustische Achse der Linse 38 und Schallachse za, zb fallen zusammen und sind von der Systemachse 34 beabstandet. Die Rotation der Stoßwellenquelle 32 kann kontinuierlich erfolgen, wobei im jeweils gleichen Zeitabständen, d. h. in vorgegebenen Positionen der Stoßwellenquelle 32 eine fokussierte Ultraschall-Stoßwelle generiert wird. Alternativ hierzu kann die Stoßwellenquelle 32 auch in unterschiedlichen diskreten Positionen vor Emission der Ultraschall-Stoßwelle angehalten werden. Auf diese Weise wird eine Abfolge von Ultraschall-Stoßwellen erzeugt, deren Foki in gleichen Winkelabständen, beispielsweise 90° auf einer Kreisbahn 39 angeordnet sind, die in einer zur Systemachse 34 senkrechten Ebene liegt. In der Fig. sind exemplarisch zwei in einem Winkelabstand von 180° erzeugte Ultraschall-Stoßwellen 2a, b eingezeichnet.

Grundsätzlich ist es auch möglich, anstelle in der Fig. 3 dargestellten Drehung der gesamten Stoßwellenquelle 32 ausschließlich die akustische Linse um die Systemachse 34 zu drehen, oder anstelle einer Drehbewegung eine Schwenkbewegung in einem begrenzten Winkelbereich durchzuführen.

Im Ausführungsbeispiel gemäß Fig. 4 ist bei ruhendem Stoßwellengenerator 36 die akustische Linse 38 mit ihrer akustischen Achse 40 unter einem Winkel α schräg zur Systemachse 34 gelagert. Die akustische Linse 38 kann dabei in verschiedenen Positionen zur Systemachse 34 derart ausgerichtet werden, dass die zu verschiedenen Positionen der akustischen Linse 38 gehörenden akustischen Achsen 40 die Einhüllende eines Kegels bilden, dessen Spitze auf der Systemachse 34 liegt. Dies kann durch Drehung der akustischen Linse 38 um die Systemachse 34 erfolgen, wie dies im Beispiel der Figur durch den Pfeil veranschaulicht ist. Bei Drehung der schräg gestellten akustischen Linse 38 um die Systemachse 34 bewegt sich deren akustische Achse 40 auf einer Kegelfläche, wobei die Kegelspitze im idealisierten Prinzipbild der Fig. 4 mit dem Punkt zusammenfällt, in dem die zu unterschiedlichen Drehpositionen der akustischen Linse 38 gehörenden akustischen Achsen 40 die Systemachse 34 schneiden. Die verschiedenen Schallachsen za, zb werden somit durch die unterschiedliche Positionierung einer deren Lage und Richtung beeinflussende Komponente erzielt, im Beispiel die akustische Linse 38, die dem ruhenden Stoßwellengenerator 36 nachgeordnet ist. Die Ultraschall-Stoßwellen 2a, b divergieren und werden in Foki Fa, Fb fokussiert, die ebenfalls idealisiert auf einer Kreisbahn 39 in einer senkrecht zur Systemachse 34 orientierter Ebene liegen.

Auch in diesem Fall kann die Verstellung der akustischen Linse 38 kontinuierlich oder diskontinuierlich erfolgen.

Die Ausrichtung (Verkippung) der akustischen Linse 38 mit unterschiedlich zur Systemachse 34 angeordneten akustischen Achsen 40 kann auch mit Hilfe von Piezoaktoren erfolgen, die in vorgegebenen Winkelpositionen um die Systemachse 34 angeordnet sind. Mit solchen Piezoaktoren ist es außerdem grundsätzlich möglich, den Kippwinkel α zu variieren und auf diese Weise Nutzbereiche mit unterschiedlicher Fokalbreite zu erzeugen.

Außerdem ist es auch möglich, nicht nur die akustische Linse sondern die gesamte Stoßwellenquelle 32 relativ zur Systemachse 34 zu verkippen.

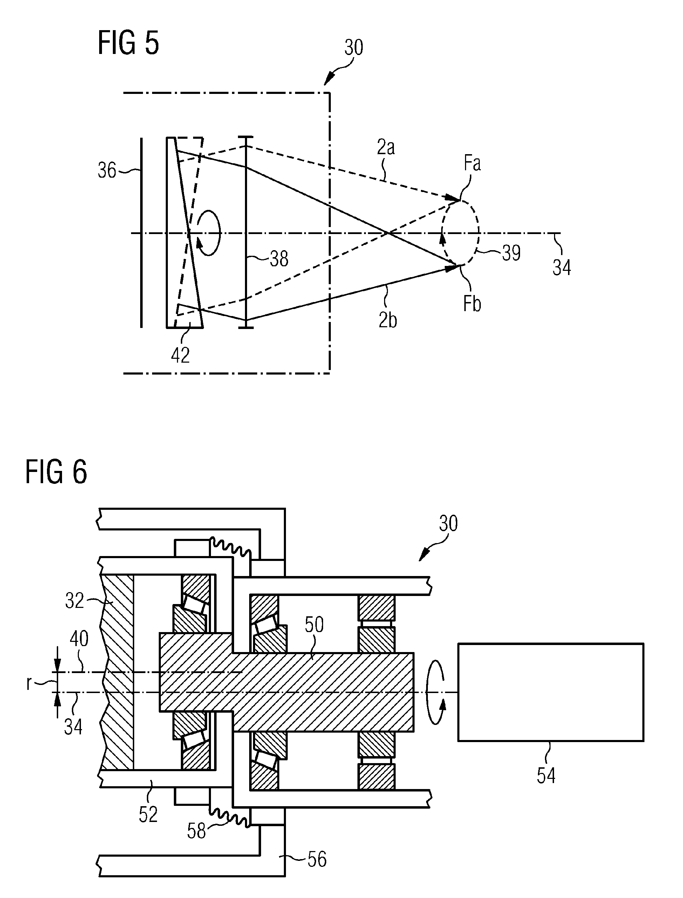

In der alternativen Ausgestaltung gemäß Fig. 5 ist als eine die Lage und Richtung der Ultraschall-Stoßwelle beeinflussende Komponente zwischen dem ruhenden Stoßwellengenerator 36 und der ebenfalls ruhenden akustischen Linse 38 eine Keilscheibe 42 angeordnet, die aus einem Werkstoff besteht, in dem die Schallgeschwindigkeit größer ist als die Schallgeschwindigkeit innerhalb des zwischen Stoßwellengenerator 34 und akustischer Linse 38 vorhandenen akustischen Koppelmediums, in der Regel Wasser. Bei einer Drehung dieser Keilscheibe 42 um die Systemachse 34, die mit der akustischen Achse der in diesem Fall ruhenden akustischen Linse 38 zusammenfällt, bewegt sich der Fokus F ebenfalls wie im vorausgegangenen Ausführungsbeispielen in einer Ebene senkrecht zur Systemachse 34 auf einer Kreisbahn 39.

Die anhand von Fig. 3 erläuterte Vorgehensweise, bei der die gesamte Stoßwellenquelle innerhalb des Stoßwellenkopfes um eine von der akustischen Achse beabstandete Systemachse gedreht wird, ist im Ausführungsbeispiel gemäß Fig. 6 näher veranschaulicht. Am freien Ende einer gekröpften Welle 50 ist drehbar ein Gehäuse 52 gelagert, in dem die Stoßwellenquelle 32 fixiert ist. Das andere Ende der gekröpften Welle 50 ist an einen Drehantrieb 54 angeschlossen, mit dem die Welle 50 um die Systemachse 34 gedreht wird. Die akustische Achse 40 der Stoßwellenquelle 32 weist einen durch die Kröpfung der Welle 50 entstandenen Abstand r zur Systemachse 34 auf. Das dem Drehantrieb 54 zugewandte Ende der Welle 50 ist drehbar im feststehenden Gehäuse 56 des ruhenden Stoßwellenkopfes 30 gelagert. Eine Drehbewegung der Welle 50 bewirkt nun eine Drehung des Gehäuses 52 um die Systemachse 34. Ein zwischen den Gehäusen 52 und 56 angeordneter, ringförmig umlaufender Faltenbalg 58 verhindert eine Drehbewegung des Gehäuses 52 um die akustische Achse 40. Der Fokus der Ultraschall-Stoßwelle beschreibt dann eine Kreisbewegung um die Systemachse 34 mit dem Radius r.

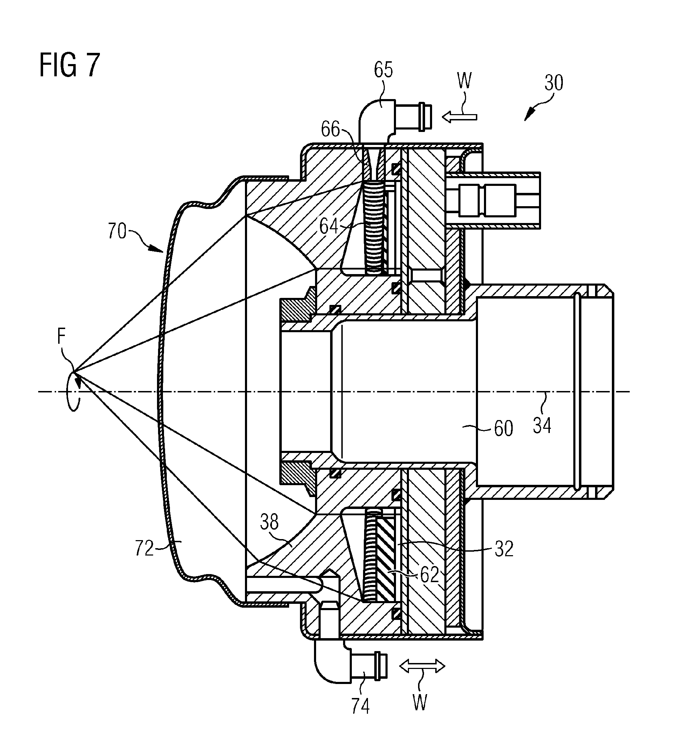

Im Ausführungsbeispiel gemäß Fig. 7 ist ein Stoßwellenkopf 30 veranschaulicht, der in einer Lithotripsie-Einrichtung mit einer sogenannten "Inline"-Röntgenortungseinrichtung zum Einsatz gelangt. In diesem Falle ist im Stoßwellenkopf 30 eine zentrale Öffnung 60 vorgesehen, durch die Röntgenstrahlen hindurchtreten können, um von einem hinter dem Stoßwellenkopf 30 angeordneten, in der Figur nicht dargestellten Röntgenempfänger empfangen zu werden. Zwischen der in diesem Fall ringförmigen elektromagnetischen Stoßwellenquelle 32 und der akustischen Linse 38 ist als die Lage und Richtung der Schallachse der Ultraschall-Stoßwellen beeinflussende Komponente ein Keilring 62 aus PVDF angeordnet, der um die Systemachse 34 des Stoßwellenkopfes 30 gedreht werden kann. An seinem äußeren Rand ist der Keilring 62 mit einem sogenannten Repellerkranz 64 versehen. Ein Zulauf 65 für Wasser W ist an eine Düse 66 angeschlossen, mit der auf den Repellerkranz 64 ein Wasserstrahl gerichtet wird, der den Keilring 62 in eine Drehbewegung versetzt. Diese Drehbewegung verursacht eine Drehbewegung des Fokus F um die Systemachse 34. Das in den zwischen Stoßwellengenerator 36 und akustischer Linse 38 befindlichen und den Keilring aufnehmenden Zwischenraum eingespeiste Wasser W dient zugleich zur Kühlung des Stoßwellengenerators 36. Am Umfang der Linse 38 ist ein Koppelbalg 70 angeordnet, mit dem der Stoßwellenkopf 30 an die Körperoberfläche eines Patienten angekoppelt werden kann. Zwischen dem Koppelbalg 70 und der akustischen Linse 38 befindet sich ein Vorlaufvolumen 72, in das oder aus dem über einen Anschlussstutzen 74 Wasser W zu- oder abgeführt werden kann.

## Patentansprüche

1. Verfahren zum Erzeugen von Ultraschall-Stoßwellen (2a-d) mit einem Stoßwellenkopf (30), in dem eine Stoßwellenquelle (32) angeordnet ist, mit der zeitlich nacheinander eine Mehrzahl von jeweils in einem Fokus (Fa-d) fokussierten Ultraschall-Stoßwellen (2a-d) erzeugt werden, die sich entlang unterschiedlicher Schallachsen (za-d) ausbreiten und eine den jeweiligen Fokus (Fa-d) umgebende Nutzzone (8a-d) mit einer Fokalbreite (B) aufweisen, wobei die Foki (Fa-d) voneinander beabstandet in vorgegebenen Positionen derart angeordnet sind, dass sich die Fokusvolumina (8a-d) der Ultraschall-Stoßwellen (2a-d) im Bereich der Nutzzonen (8a-d) derart überlagern, dass sich ein zusammenhängender Nutzbereich (20) ergibt, dessen effektive Fokalbreite (ΣB) größer ist als die Fokalbreite (B) der Nutzzonen (8a-d) der einzelnen Ultraschall-Stoßwellen (2a-d).

2. Verfahren nach Anspruch 1 oder 2, bei dem die Schallachsen (za-d) symmetrisch um eine Systemachse (34) des Stoßwellenkopfes (30) angeordnet sind.

3. Verfahren nach Anspruch 1 oder 2, bei dem sich die Foki (Fa-d) der Ultraschall-Stoßwellen (2a-d) zumindest annähernd in einer zur Systemachse (34) senkrechten Ebene befinden.

4. Verfahren nach Anspruch 3, bei dem die Foki (Fa-d) auf einer Kreisbahn (39) um die Systemachse (34) angeordnet sind.

5. Verfahren nach einem der Ansprüche 1 bis 4, bei dem die Schallachsen (za-d) zueinander parallel sind.

6. Verfahren nach einem der Ansprüche 1 bis 4, bei dem die Schallachsen (za-d) zueinander geneigt sind.

7. Verfahren nach Anspruch 6, bei dem die Schallachsen (za-d) derart angeordnet sind, dass die Ausbreitungsrichtungen der Ultraschall-Stoßwellen (2a-d) divergieren.

8. Verfahren nach einem der vorhergehenden Ansprüche, bei dem zum Erzeugen der entlang verschiedener Schallachsen (za-d) sich ausbreitenden Ultraschall-Stoßwellen (2a-d) die Stoßwellenquelle (32) innerhalb des ruhenden Stoßwellenkopfes (30) unterschiedlich positioniert wird.

9. Verfahren nach einem der Ansprüche 1 bis 7, bei dem die Stoßwellenquelle (32) einen Stoßwellengenerator (36) sowie eine diesem nachgeordnete, die Lage und Richtung der Schallachse (za-d) der Ultraschall-Stoßwellen (2a-d) beeinflussende Komponente (38,42,62) umfasst, die zum Erzeugen der entlang verschiedener Schallachsen (za-d) sich ausbreitenden Ultraschall-Stoßwellen (2a-d) relativ zum ruhenden Stoßwellengenerator (36) unterschiedlich positioniert werden.

10. Stoßwellenkopf (30) zum Durchführen eines Verfahren nach einem der vorhergehenden Ansprüche, mit einer darin angeordneten Stoßwellenquelle (32) zum Erzeugen von fokussierten Ultraschall-Stoßwellen (2a-d), die sich bei ruhendem Stoßwellenkopf (30) entlang unterschiedlicher Schallachsen (za-d) ausbreiten, und deren Foki (Fa-d) voneinander beabstandet sind, und die eine den jeweiligen Fokus (Fa-d) umgebende Nutzzone (8a-d) mit einer Fokalbreite (B) aufweisen, wobei sich die Fokusvolumina (6a-d) der Ultraschall-Stoßwellen (2a-d) im Bereich der Nutzzonen (8a-d) derart überlagern, dass sich ein zusammenhängender Nutzbereich (20) ergibt, dessen Fokalbreite (ΣB) größer ist als die Fokalbreite (B) der einzelnen Ultraschall-Stoßwellen (2a-d).

11. Stoßwellenkopf (30) nach Anspruch 10, bei dem die Schallachsen (za-d) symmetrisch um eine Systemachse (34) angeordnet sind.

12. Stoßwellenkopf (30) nach Anspruch 10 oder 11, bei dem sich die Foki (Fa-d) der Ultraschall-Stoßwellen (2a-d) zumindest annähernd in einer zur Systemachse (34) senkrechten Ebene befinden.

13. Stoßwellenkopf (30) nach Anspruch 12, bei dem die Foki (Fa-d) auf einer Kreisbahn (39) um die Systemachse (34) angeordnet sind.

14. Stoßwellenkopf (30) nach einem der Ansprüche 10 bis 13, bei dem die Schallachsen (za-d) zueinander geneigt sind.

15. Stoßwellenkopf (30) nach Anspruch 14, bei dem die Schallachsen (za-d) derart angeordnet sind, dass die Ausbreitungsrichtungen der Ultraschall-Stoßwellen (2a-d) divergieren.

16. Stoßwellenkopf (30) nach einem der Ansprüche 10 bis 15, bei dem die Stoßwellenquelle (32) innerhalb des Stoßwellenkopfes (30) variabel positionierbar ist.

17. Stoßwellenkopf (30) nach Anspruch 16, bei dem die Stoßwellenquelle (32) schwenkbar um eine Systemachse (34) im Stoßwellenkopf (30) angeordnet ist.

18. Stoßwellenkopf (30) nach einem der Ansprüche 10 bis 15, bei dem die Stoßwellenquelle (32) einen Stoßwellengenerator (36) sowie diesem nachgeordnete, die Lage und Richtung der Schallachse der Ultraschall-Stoßwellen beeinflussende Komponente (38,42,62) umfasst, die zum Erzeugen der entlang verschiedener Schallachsen (za-d) sich ausbreitenden Ultraschall-Stoßwellen (2a-d) relativ zum ruhenden Stoßwellengenerator (36) variabel positionierbar sind.

19. Stoßwellenkopf (30) nach Anspruch 18, bei dem als die Lage und Richtung der Schallachse (za-d) der Ultraschall-Stoßwellen beeinflussende Komponente eine schwenkbar um eine Systemachse gelagerte akustische Linse (38) vorgesehen ist.

20. Stoßwellenkopf (30) nach Anspruch 19, bei dem die akustische Achse (40) der akustischen Linse (38) parallel und beabstandet zur Systemachse (34) angeordnet ist.

21. Stoßwellenkopf (30) nach Anspruch 19, bei dem die akustische Achse (40) der akustischen Linse (38) geneigt zur Systemachse verläuft.

22. Stoßwellenkopf (30) nach Anspruch 18, bei dem als die Lage und Richtung der Schallachse (za-d) der die Ultraschall-Stoßwellen (2a-d) beeinflussenden Komponente zwischen einer akustischen Linse (38) und einem Stoßwellengenerator (36) mit ebener Abstrahlfläche schwenkbar um eine Systemachse (34) eine oder ein mit ihren bzw. seinen Planflächen an ein Koppelmedium angrenzende Keilscheibe (42) bzw. Keilring (62) angeordnet ist, die aus einem Werkstoff bestehen, in dem die Schallgeschwindigkeit größer als die Schallgeschwindigkeit im Koppelmedium ist.
